# EUROPEAN PATENT APPLICATION

(11) **EP 4 382 092 A1**
(43) Date of publication of application: **12.06.2024**
(21) Application number: 23809941.0
(22) Date of filing: 27.10.2023
(51) Int. Cl.: A61K 9/08, A61K 47/18, A61K 47/26, A61K 47/42, A61K 38/48, C12N 9/52

(54) **STABLE LIQUID FORMULATION CONTAINING BOTULINUM TOXIN**

(30) Priority: 28.10.2022 KR 20220141776
(71) Applicant: Pharma Research Bio Co., Ltd., Gangneung-si, Gangwon-do 25451 (KR)
(72) Inventor: The designation of the inventor has not yet been filed
(74) Representative: Cabinet Beau de Loménie
(86) International application number: PCT/KR2023/016817
(87) International publication number: WO 2024/091049

(57) **Abstract**

Provided are a liquid formulation including botulinum toxin and proline and a preparation method thereof, and a use thereof.

## Description

### BACKGROUND OF THE INVENTION

### 1. Field of the Invention

The present disclosure relates to a liquid formulation including botulinum toxin and proline and a preparation method thereof, and a use thereof.

### 2. Description of the Related Art

Botulinum toxin is a type of neurotoxic protein produced by bacteria such as *Clostridium botulinum,* and binds irreversibly to the presynaptic nerve terminals, which results in the inhibition of acetylcholine release in synapses, thereby blocking muscle contraction and exhibiting the secondary effect of muscle relaxation. Due to these functions, botulinum toxin has been used for therapeutic and cosmetic purposes since approval by the U.S. FDA in 1989 (KR 10-2010-0107475 A, KR 10-2008-0049152 A, *etc.).*

Botulinum toxin is used for therapeutic purposes in neuromuscular diseases such as strabismus, torticollis, or blepharospasm, and for cosmetic purposes in removing wrinkles or frown lines, and treating square jaw, hyperhidrosis, or migraine as an injectable formulation. As side effects, cases such as dysphagia, voice change, dry mouth, blurred vision, *etc.* have been reported, but thus far there have been no direct reports of deaths caused by botulinum toxin. Therefore, botulinum toxin is evaluated as being a very safe drug when used appropriately.

However, protein agents such as botulinum toxin have many problems during their formulation process. This is attributable to instability of the protein, and the problem is serious in the case of protein agents such as botulinum toxin, which are formulated at a very low concentration.

Commercial botulinum toxin formulations are traditionally prepared as a freeze-dried or vacuum-dried dry powder in order to increase stability of the protein. However, dry powder formulations prepared by methods such as freeze-drying, *etc.* must undergo a rehydration process before use, and thus there is inconvenience in use. Further, due to errors in the dilution process during the rehydration process, there is a problem of deviations in the administration amount of botulinum toxin, in particular, deviations in terms of the activity. For these reasons, there is a growing demand for liquid formulations capable of providing a user with convenience and minimizing errors in the rehydration process.

Since development of a stable liquid formulation that may be stored for a long period of time while maintaining the activity of botulinum toxin for a long time is still insufficient, it is necessary to develop a new composition of a liquid formulation including botulinum toxin.

### SUMMARY OF THE INVENTION

An object of the present disclosure is to provide a liquid formulation including botulinum toxin and proline.

Another object of the present disclosure is to provide a method of preparing the liquid formulation.

Still another object of the present disclosure is to provide a method of stabilizing botulinum toxin using the liquid formulation.

### BRIEF DESCRIPTION OF THE DRAWINGS

FIG. 1 shows results of evaluating the residual potency according to each type of excipient;
FIG. 2 shows results of evaluating the residual potency according to combinations of excipient and disaccharide;
FIG. 3 shows results of evaluating the residual potency according to concentrations of proline and sucrose;
FIG. 4 shows results of evaluating the residual potency according to combinations of excipient and albumin;
FIG. 5 shows results of evaluating the residual potency according to combinations of excipient, disaccharide, and albumin; and
FIG. 6 shows results of evaluating the residual potency according to concentrations of proline and sucrose in liquid formulations including albumin.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

One aspect of the present disclosure provides a liquid formulation including botulinum toxin and proline.

Specifically, the liquid formulation may be characterized by including botulinum toxin and proline; or characterized by including botulinum toxin, sucrose, and proline; or characterized by including botulinum toxin, albumin, and proline; or characterized by including botulinum toxin, sucrose, albumin, and proline; but is not limited thereto. Further, the liquid formulation of the present disclosure may further include an isotonic agent, a non-ionic surfactant, a buffer, or a combination thereof, but is not limited thereto.

As used herein, the term "liquid formulation" means a drug which is formulated into a liquid form, which includes both liquid formulations for internal use and external use.

The liquid formulation of the present disclosure includes substances capable of stably maintaining and/or storing botulinum toxin, which exhibits pharmacological effects, for a predetermined period of time when formulated into a liquid form. Ingredients other than botulinum toxin exhibiting pharmacological effects of the liquid formulation may be used interchangeably with a stabilizer. As used herein, the term "stabilizer" refers to a substance that stably maintains components such as active ingredients, *etc.* in a formulation for a predetermined period of time.

The liquid formulation of the present disclosure is characterized by including a composition of the stabilizer capable of stabilizing botulinum toxin to maintain its pharmacological effects for a long time even though stored for a long period of time.

As used herein, the "botulinum toxin" is also referred to as *"Clostridium botulinum* toxin", a type of proteins derived from *Clostridium botulinum,* and refers to a protein that binds irreversibly to the presynaptic nerve terminals, which results in the inhibition of acetylcholine release in synapses, thereby blocking muscle contraction and exhibiting the secondary effect of muscle relaxation.

Botulinum toxin protein has a molecular weight of about 150 kDa, and is divided into 7 types from A to G according to serological characteristics. Botulinum toxin type A is the most lethal natural substance known to humans, and in addition to serotype A, six other serotypes of botulinum toxins which are generally immunologically distinct have been identified, i.e., botulinum toxin serotypes B, C, D, E, F, and G. Different serotypes may be identified by neutralization with type-specific antibodies. The different serotypes of botulinum toxin vary in the severity of the paralysis they evoke and in the animal species that they affect.

The molecular weight of the botulinum toxin molecule, for all seven of the known botulinum toxin serotypes, is about 150 kDa. Meanwhile, the botulinum toxins are released by Clostridial bacterium as complexes including the 150 kDa botulinum toxin protein molecule along with associated non-toxin proteins. Thus, the botulinum toxin type A complex may be produced by Clostridial bacterium as 900 kDa, 500 kDa and 300 kDa forms. Botulinum toxin types B and C may be produced as 500 kDa complexes, and botulinum toxin type D may be produced as 300 kDa and 500 kDa complexes. Botulinum toxin types E and F may be produced as 300 kDa complexes. These complexes (i.e., molecular weight greater than about 150 kDa) are believed to include a non-toxin hemagglutinin protein and a non-toxin and non-toxic non-hemagglutinin protein.

The botulinum toxin includes a high-molecular-weight complex form containing a pure neurotoxic component of about 150 kDa and non-toxin proteins. Thus, the complexed form may include the botulinum neurotoxin protein and one or more non-toxic hemagglutinin proteins, and/or one or more non-toxic non-hemagglutinin proteins. Specifically, the botulinum toxin complex protein may be a complex of botulinum neurotoxin (BoNT), non-toxic non-hemagglutinin (NTNH), and hemagglutinin (HA) protein. The molecular weight of the complex may be higher than about 150 kDa. For example, the complexed form of the botulinum toxin type A may have a molecular weight of about 900 kDa, about 500 kDa, or about 300 kDa. A method of purifying the *Clostridium botulinum* toxin complex of the present disclosure may purify the various complexes.

In one embodiment, the botulinum toxin of the present disclosure may be type A toxin.

In one embodiment of the above-described embodiments, the botulinum toxin of the present disclosure may be in the form of a complex of being bound with a non-toxic protein. Specifically, the botulinum toxin complex protein of the present disclosure may be a complex of botulinum toxin (BoNT), non-toxic non-hemagglutinin (NTNH), and HA (hemagglutinin) protein. More specifically, the botulinum toxin complex protein of the present disclosure may be a complex of BoNT, NTNH, hemagglutinin 70 (HA70), hemagglutinin 33 (HA33), and hemagglutinin 17 (HA17). More specifically, the HA70 may be divided into HA20 (hemagglutinin 20) and HA50 (hemagglutinin 50), or the BoNT may be divided into about 50 kDa light chain (LC) and about 100 kDa heavy chain (HC), but is not limited thereto.

In one embodiment of the above-described embodiments, the botulinum toxin complex protein of the present disclosure may have a molecular weight of more than 150 kDa. Specifically, it may have a molecular weight of about 250 kDa to 1400 kDa, more specifically, a molecular weight of 280 kDa to 1300 kDa, 300 kDa to 1200 kDa, 700 kDa to 1100 kDa, 800 kDa to 1000 kDa, or about 900 kDa, but is not limited thereto.

The botulinum toxin of the present disclosure may include all of those obtained from *Clostridium botulinum* culture or those commercially available, and the botulinum toxin may be included in a final amount of 100 units/mL, but is not limited thereto.

As used herein, the term "proline" is one of amino acids and serves to help maintain the pharmacological effects of botulinum toxin for a long time with respect to the objects of the present disclosure.

The liquid formulation of the present disclosure may include proline at a concentration of about 0.01% (w/v) to about 0.5% (w/v), about 0.05% (w/v) to about 0.5% (w/v), about 0.1% (w/v) to about 0.5% (w/v), about 0.1% (w/v), about 0.2% (w/v), about 0.3% (w/v), about 0.4% (w/v), or about 0.5% (w/v), but is not limited thereto.

For example, it was confirmed that the liquid formulation of the present disclosure has the most excellent effect of maintaining the activity of botulinum toxin when proline among various excipients is added.

The liquid formulation according to the present disclosure may further include sucrose, but is not limited thereto. The sucrose may increase the stability of botulinum toxin in the liquid formulation.

The liquid formulation of the present disclosure may include sucrose at a concentration of about 0.5% (w/v) to about 20% (w/v), about 0.5% (w/v) to about 15% (w/v), about 0.5% (w/v) to about 10% (w/v), about 0.5% (w/v) to about 8% (w/v), about 0.5% (w/v) to about 5% (w/v), about 0.5% (w/v) to about 4% (w/v), about 1% (w/v) to about 20% (w/v), about 1% (w/v) to about 15% (w/v), about 1% (w/v) to about 10% (w/v), about 1% (w/v) to about 8% (w/v), about 1% (w/v) to about 6% (w/v), or about 0.0% (w/v), about 1.0% (w/v), about 2.0% (w/v), about 3.0% (w/v), about 4.0% (w/v), about 5.0% (w/v), about 6.0% (w/v), or about 8.0% (w/v), but is not particularly limited thereto.

For example, it was confirmed that the liquid formulation of the present disclosure shows a residual potency of 90% or more for 10 weeks under 37°C accelerated conditions when sucrose is further added in addition to proline. This suggests that the combination of proline and sucrose in the liquid formulation including botulinum toxin may serve as a stabilizer to preserve botulinum toxin for a long period of time without a decrease in potency in the liquid formulation.

The liquid formulation according to the present disclosure may further include albumin. The albumin is a type of protein, and is known as a plasma protein that transports various substances including metal ions, drugs, and xenobiotics, and is known to be related to pH control and osmotic pressure maintenance in the culture environment. In the present disclosure, the albumin may be, but is not limited to, those obtained by extraction from human plasma or serum (Human Serum Albumin; HSA) or those obtained by recombination (Recombinant Human Serum Albumin; rHSA). Specifically, the albumin may be animal-derived HSA or plant-derived recombinant HSA, but is not limited thereto. The recombinant albumin has an advantage of low immunogenicity, because blood-borne contaminants are not included, and only defined substances may be used.

The liquid formulation of the present disclosure may include the albumin at a concentration of about 0.01% (w/v) to about 1% (w/v), about 0.05% (w/v) to about 1% (w/v), about 0.1% (w/v) to about 1% (w/v), about 0.1% (w/v) to about 0.8% (w/v), about 0.2% (w/v) to about 0.7% (w/v), about 0.3% (w/v) to about 0.6% (w/v), about 0.3% (w/v), about 0.4% (w/v), about 0.5% (w/v), or about 0.6% (w/v), but is not limited thereto.

A specific example of the liquid formulation according to the present disclosure may include a liquid formulation including botulinum toxin and proline, and a more specific example thereof may include a liquid formulation including 100 units/mL botulinum toxin and 0.01% (w/v) to 0.5% (w/v) proline.

The liquid formulation according to the present disclosure may further include sucrose. A specific example thereof may include a liquid formulation including botulinum toxin, sucrose, and proline. A more specific example thereof may include a liquid formulation including 100 units/mL botulinum toxin, 0.5% (w/v) to 20% (w/v) sucrose, and 0.01% (w/v) to 0.5% (w/v) proline.

The liquid formulation according to the present disclosure may further include albumin. A specific example thereof may include a liquid formulation including botulinum toxin, albumin, and proline. A more specific example thereof may include a liquid formulation including 100 units/mL botulinum toxin, 0.01% (w/v) to 1% (w/v) albumin, and 0.01% (w/v) to 0.5% (w/v) proline.

Another specific example thereof may include a liquid formulation including botulinum toxin, sucrose, albumin, and proline. More specific example thereof may include a liquid formulation including 100 units/mL botulinum toxin, 0.5% (w/v) to 20% (w/v) sucrose, 0.01% (w/v) to 1% (w/v) albumin, and 0.01% (w/v) to 0.5% (w/v) proline, but the liquid formulation of the present disclosure is not limited to the above examples.

The liquid formulation of the present disclosure may further include a non-ionic surfactant and/or an isotonic agent.

The non-ionic surfactant may lower surface tension of the protein solution to prevent adsorption or aggregation of proteins on the hydrophobic surface.

Specific examples of the non-ionic surfactant which may be used in the present disclosure may include polysorbates (e.g., polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate); the number (20) following the polyoxyethylene refers to the total number of oxyethylene group (-CH₂CH₂O-)), poloxamer (PEO-PPO-PEO copolymer; PEO: poly(ethylene oxide), PPO: polypropylene oxide)), polyethylene-polypropylene glycol, polyoxyethylene compounds (e.g., polyoxyethylene stearate, polyoxyethylene alkyl ether (alkyl: C₁-C₃₀), polyoxyethylene monoallyl ether, alkylphenyl polyoxyethylene copolymer (alkyl: C₁-C₃₀), *etc.),* sodium dodecyl sulfate (SDS), *etc.,* or polysorbate or poloxamer, which may also be used in a combination of two or more thereof.

Specifically, the non-ionic surfactant may be poloxamer 188, polysorbate 20, polysorbate 40, polysorbate 60, or polysorbate 80, which may be used in combination, but is not particularly limited thereto.

In the present disclosure, the non-ionic surfactant may be included at a concentration of about 0.2% (w/v) or less, for example, about 0.001% (w/v) to about 0.2% (w/v), about 0.001% (w/v) to about 0.1% (w/v), about 0.005% (w/v) to about 0.1% (w/v), about 0.001% (w/v) to about 0.08% (w/v), about 0.002% (w/v) to about 0.08% (w/v), about 0.005% (w/v) to about 0.05% (w/v), about 0.01% (w/v) to about 0.05% (w/v), or about 0.05% (w/v) in the liquid formulation of the present disclosure, but is not particularly limited thereto.

The isotonic agent is a substance added to adjust the osmotic pressure of the liquid formulation, and may be included without limitation in the liquid formulation of the present disclosure as long as it may contribute to stabilizing botulinum toxin while maintaining an appropriate osmotic pressure.

The isotonic agent may include a water-soluble inorganic salt, and specifically, the liquid formulation of the present disclosure may include, as the isotonic agent, one or more selected from the group consisting of sodium chloride, glycerin, potassium chloride, and dextrose, but is not limited thereto.

In the present disclosure, the isotonic agent may be included at a concentration of about 5% (w/v) or less, for example, about 0.01% (w/v) to about 5% (w/v), about 0.1% (w/v) to about 5% (w/v), about 0.1% (w/v) to about 3% (w/v), about 0.1% (w/v) to about 2% (w/v), about 0.5% (w/v) to about 1.5% (w/v), about 0.75% (w/v) to about 1.25% (w/v) or about 0.9% (w/v) in the liquid formulation of the present disclosure, but is not particularly limited thereto.

The liquid formulation of the present disclosure may further include a buffer. For example, the buffer may be one or more selected from the group consisting of acetic acid and salts thereof, citric acid and salts thereof, and phosphoric acid and salts thereof, for example, phosphoric acid and salts thereof (e.g., phosphate, sodium phosphate, potassium phosphate, or hydrogen or dihydrogen salts thereof), citric acid and salts thereof (e.g., sodium citrate), acetic acid and salts thereof (e.g., sodium acetate), or sodium phosphate, but is not limited thereto.

In the present disclosure, the buffer may be included at a concentration of about 5.0% (w/v) or less, at a concentration of about 4.0% (w/v) or less, at a concentration of about 3.0% (w/v) or less, at a concentration of about 2.0% (w/v) or less, at a concentration of about 1.0% (w/v) or less, at a concentration of about 0.9% (w/v) or less, at a concentration of about 0.8% (w/v) or less, for example, about 0.01% (w/v) to about 5.0% (w/v), about 0.01% (w/v) to about 4.0% (w/v), about 0.01% (w/v) to about 3.0% (w/v), about 0.01% (w/v) to about 2.0% (w/v), about 0.01% (w/v) to about 1.0% (w/v), about 0.01% (w/v) to about 5.0% (w/v), about 0.1% (w/v) to about 1.0% (w/v), about 0.01% (w/v) to about 0.9% (w/v), about 0.05% (w/v) to about 0.9% (w/v), about 0.1% (w/v) to about 1.00% (w/v), about 0.4% (w/v) to about 0.9% (w/v), about 0.4% (w/v) to about 2.0% (w/v), about 0.3% (w/v) to about 1.0% (w/v), about 0.3% (w/v) to about 0.8% (w/v), or about 0.05% (w/v), or about 0.7% (w/v) in the liquid formulation of the present disclosure, but is not particularly limited thereto.

The liquid formulation of the present disclosure may include a pharmaceutically acceptable salt form.

The term "pharmaceutically acceptable" refers to a substance that may be effectively used for a desired purpose without causing excessive toxicity, irritation, or allergic responses within the scope of medical judgment.

As used herein, the "pharmaceutically acceptable salt" includes salts derived from pharmaceutically acceptable inorganic or organic acids or bases. Examples of suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Salts derived from suitable bases may include alkali metals, such as sodium or potassium, *etc.,* alkaline earth metals such as magnesium, *etc.,* ammonium, *etc.*

Further, the liquid formulation according to the present disclosure may further include a pharmaceutically acceptable carrier, excipient, *etc.,* and such a carrier or excipient may exist non-naturally.

Specifically, the "pharmaceutically acceptable carrier" refers to a carrier or diluent that does not cause significant irritation to an organism and does not abrogate the properties of the liquid formulation of the present disclosure. In a composition formulated into a liquid solution, the pharmaceutically acceptable carrier may include saline, sterile water, Ringer's solution, buffered saline, an albumin injectable solution, a dextrose solution, a maltodextrin solution, glycerol, and ethanol, which are sterile and biocompatible, and a mixture of one or more components thereof. As needed, other common additives, including an antioxidant, a buffer, a bacteriostatic agent, etc. may be added thereto.

More specifically, carriers, excipients, and diluents that may be included in the pharmaceutical composition may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, polycaprolactone, polylactic acid, poly-L-lactic acid, mineral oil, *etc.*

The liquid formulation of the present disclosure may be used for the treatment or prevention of diseases that may be prevented, treated, or improved by administration of botulinum toxin, for example, for the treatment or prevention of neuromuscular disorders characterized by skeletal muscle hyperactivity. Further, botulinum toxin may be used to treat various diseases, such as headaches, migraine headaches, tension headaches, sinus headaches, cervicogenic headaches, sweating disorders, axillary hyperhidrosis, palmar hyperhidrosis, plantar hyperhidrosis, Frey's syndrome, hyperkinetic skin lines, facial wrinkle, glabellar lines, crow's feet, marionette lines, a nasolabial fold, a skin disorder, achalasia, strabismus, chronic anal fissure, blepharospasm, musculoskeletal pain, fibromyalgia, pancreatitis, tachycardia, prostatic enlargement, prostatitis, urinary retention, urinary incontinence, overactive bladder, hemifacial spasm, tremors, myoclonus, gastrointestinal disorders, diabetes, sialorrhea, detrusor-sphincter dyssynergia, post stroke spasticity, wound healing, juvenile cerebral palsy, smooth muscle spasm, restenosis, a focal dystonia, epilepsy, cervical dystonia, thyroid disorder, hypercalcemia, an obsessive compulsive disorder, arthritic pain, Raynaud's syndrome, striae distensae, peritoneal adhesion, vasospasms, rhinorrhea, muscle contracture, laryngeal dystonia, writer's cramp, carpel tunnel syndrome, *etc.,* and used for cosmetic purposes, but is not limited thereto.

As used herein, the term "prevention" means all of the actions by which diseases are restrained by administration of botulinum toxin or the liquid formulation including the same or the occurrence of diseases is restrained or retarded by administration of the liquid formulation. As used herein, the term "treatment" means all of the actions by which symptoms of target diseases have taken a turn for the better or been modified favorably by administration of botulinum toxin or the liquid formulation including the same.

In specific embodiments of the present disclosure, the liquid formulation of the present disclosure may be used for the treatment or prevention of neuromuscular disorder, but is not limited thereto.

Further, the liquid formulation of the present disclosure may have a formulation and composition suitable for subcutaneous administration, and thus may be administered by a route of subcutaneous administration, but is not limited thereto.

As used herein, the term "administration" means introducing the liquid formulation into a patient by any appropriate method, and may be, for example, intramuscular administration or subcutaneous administration, but is not limited thereto.

The total administration dosage of the liquid formulation of the present disclosure may be about 0.0001 mg to 500 mg per 1 kg of a patient's body weight a day, and the liquid formulation may be formulated such that it may be appropriately administered in the above dosage. However, with regard to the dosage of botulinum toxin, its effective administration dosage for a patient is determined by considering various factors such as the patient's age, body weight, health condition, sex, severity of disease, diet and excretion rate, as well as the route of administration and number of treatments. Considering this point, those skilled in the art will be able to determine an appropriate effective dosage according to the specific use of the liquid formulation of the present disclosure.

As used herein, the term "about" includes the exact number following the term as well as approximately the number or ranges near to the number. Considering the context in which the number is presented, whether a number is near to or approximately a specifically recited number may be determined. For example, the term "about" may refer to a range of -10% to +10% of a numeric value. As another example, the term "about" may refer to a range of -5% to +5% of a given numerical value. However, it is not limited thereto.

Another aspect of the present disclosure provides a method of preparing the liquid formulation.

Specifically, the preparation method may include the step of mixing botulinum toxin and proline with each other, and may further include the step of additionally mixing with albumin, sucrose, or both of them, but is not limited thereto.

Still another aspect of the present disclosure provides a method of stabilizing botulinum toxin using the liquid formulation.

The liquid formulation may further include one or more components selected from the group consisting of sucrose, albumin, isotonic agent, non-ionic surfactant, and buffer, but is not limited thereto.

The liquid formulation and components constituting the same are as described above.

Protein agents such as botulinum toxin of the present disclosure have many problems during their formulation process. This is attributable to instability of the protein, and the problem is serious in the case of protein agents such as botulinum toxin, which are formulated at a very low concentration. Because of high toxicity, botulinum toxin must be administered in a small amount for therapeutic or cosmetic purposes, and thus a liquid formulation with a low concentration of botulinum toxin is preferred over a liquid formulation with a high concentration of botulinum toxin.

In this respect, there is an urgent need for a new type of liquid formulation capable of constantly maintaining the activity of botulinum toxin. The liquid formulation of the present disclosure may maintain the activity of botulinum toxin even when stored for a long time, and also has excellent administration stability.

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Preparation of liquid formulation of botulinum toxin type A

Liquid formulations of botulinum toxin type A (molecular weight of about 900 kDa) were prepared by the following method. 50 mmol of sodium phosphate as a buffer, 0.9% sodium chloride as an isotonic agent, and 0.05% (w/v) polysorbate 20 as a surfactant were added. To the buffer including botulinum toxin type A, the isotonic agent, and the surfactant, each excipient was added at the corresponding concentration and homogeneously mixed and dissolved, and then adjusted to pH 6.0, followed by filtration using 0.22 µm. The filtered solution was used to prepare each botulinum toxin liquid formulation at a final concentration of 100 units/mL (Table 1).

**[Table 1]**

| Basic composition | Content |
|---|---|
| Botulinum toxin type A | 100 units/mL |
| Sodium phosphate | 0.7% (w/v) |
| Sodium chloride | 0.9% (w/v) |
| Polysorbate 20 | 0.05% (w/v) |

The excipient candidates were added at the concentrations shown in Table 2 below. The following excipient candidates were added by adjusting their concentration, based on the maximum content of additives announced by the Ministry of Food and Drug Safety.

**[Table 2]**

| Excipient candidate of botulinum toxin | Excipient concentration |
|---|---|
| Human serum albumin | 0.5% (w/v) |
| Meglumine+Glutamic acid | 5% (w/v), 3% (w/v) |
| *N*-Acetylcysteine | 0.2% (w/v) |
| Proline | 0.2% (w/v) |
| Taurine | 1% (w/v) |
| Glutathione | 1% (w/v) |
| PEG3350 | 1% (w/v) |

### Example 2. Evaluation of stability of botulinum toxin type A

To evaluate stability of the botulinum toxin liquid formulation prepared in Example 1, ELISA method using an anti-SNAP25 antibody was used to examine the effect on SNAP25 cleavage under 37°C accelerated conditions, and through this, the toxin potency was calculated. The prepared botulinum toxin liquid formulations were stored under 37°C accelerated conditions and sampled at regular intervals (every 1 week) to examine the toxin potency changing depending on the storage time.

ELISA was performed by way of the following method. The liquid formulation to be tested and recombinant SNAP25 (abcam, USA) were mixed at a ratio of 100:1, respectively, and an enzyme reaction was allowed in a 37°C chamber. 21 hours later, the liquid formulation-SNAP25 mixture was taken out and mixed with a coating buffer (100 mM bicarbonate/carbonate, pH 9.6), to which 10 mM ethylenediaminetetraacetic acid (EDTA) was added, at a ratio of 1:1, and then 100 µL thereof was dispensed into each well of a 96-well plate (Thermo, USA), and coated for 2 hours at room temperature. After the coating was finished, each well was washed three times with a washing buffer (Thermo, USA), and then blocked using 5% skim milk for 2 hours at room temperature. Washing was then performed three times using the washing buffer, and 100 µL of a cleaved SNAP25-specific antibody (mybiosource, USA) diluted in 5% skim milk was dispensed into each well and allowed to react at room temperature for 2 hours. Thereafter, washing was performed three times using the washing buffer and allowed to react with HRP-conjugated secondary antibody (abcam, USA) diluted in 5% skim milk at room temperature for 2 hours. After the reaction, washing was performed three times using the washing buffer, and color development was induced by dispensing 100 µL of TMB substrate solution (Thermo, USA) to each well. The reaction was stopped by adding the equal amount of 2 N sulfuric acid (Sigma, USA), and then absorbance at 450 nm was measured using an absorbance reader (BioTek, USA), and the measured values were expressed as the toxin potency in % using the values of positive and negative controls. Toxin potency was calculated by the following formula.

Toxin potency (%) = (Color development value of a liquid formulation experimental group to be tested - Color development value of an experimental group not treated with toxin) / (Color development value of an experimental group treated with only BoNT/A stock solution at a concentration of 100 units/mL - Color development value of an experimental group not treated with toxin) × 100

### Example 3. Evaluation of stabilizer of botulinum toxin type A liquid formulation

For the liquid formulations of botulinum toxin type A of Example 1, an experiment was performed to evaluate the potency at 1-week intervals under 37°C accelerated conditions according to the method of Example 2.

### 3-1. Evaluation of residual potency according to type of excipient

Residual potency according to the type of excipient was evaluated. In detail, meglumine+glutamic acid, N-acetylcysteine, proline, taurine, glutathione, or PEG3350 was used as the excipient, and the concentration of the excipient included in the botulinum toxin liquid formulation composition is shown in Table 2.

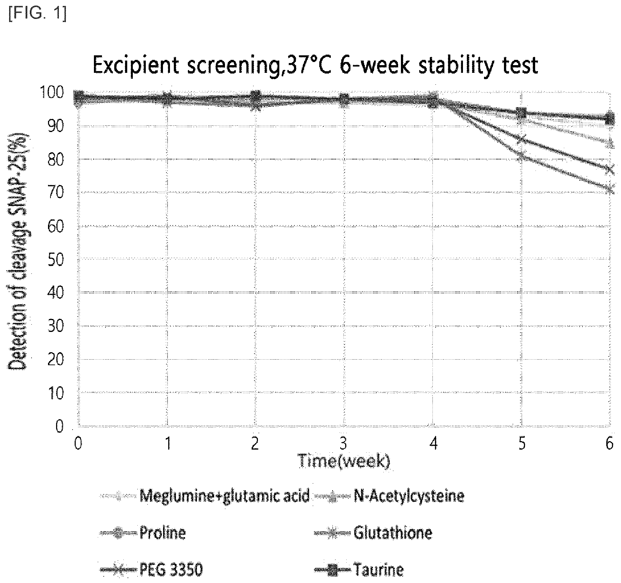

As a result, as shown in FIG. 1, it was confirmed that among the excipients, proline maintained a residual potency of 90% or more for 6 weeks or more, and it was found that the excipient showed the most excellent effect of maintaining the activity of botulinum toxin.

### 3-2. Evaluation of residual potency according to combination of disaccharide

In order to examine whether the residual potency was excellent even when disaccharide was added, disaccharide was further included in addition to the excipient of Example 3-1 to evaluate the residual potency.

In detail, sucrose, lactose, and combinations thereof were used as the disaccharide.

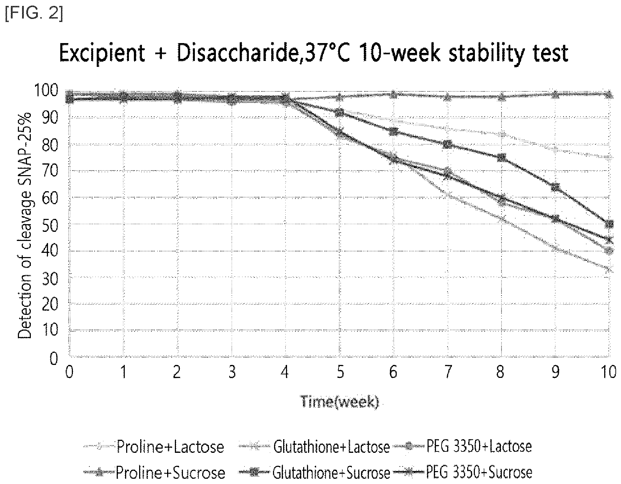

As a result, as shown in FIG. 2, it was confirmed that combination of proline and sucrose showed the most excellent residual potency of 90% or more for 10 weeks under 37°C accelerated conditions.

The above results reaffirmed that the combination of proline and sucrose as a stabilizer in the composition including botulinum toxin may preserve botulinum toxin for a long period of time without reducing its potency in the liquid formulation.

### 3-3. Evaluation of residual potency according to concentration of sucrose

Example 3-2 confirmed that the combination of proline and sucrose was most effective as the stabilizer, and the residual potency was evaluated according to the concentration of proline and sucrose.

In detail, 0.1% (w/v) to 0.3% (w/v) proline was used, and 0% (w/v) to 8% (w/v) sucrose was used.

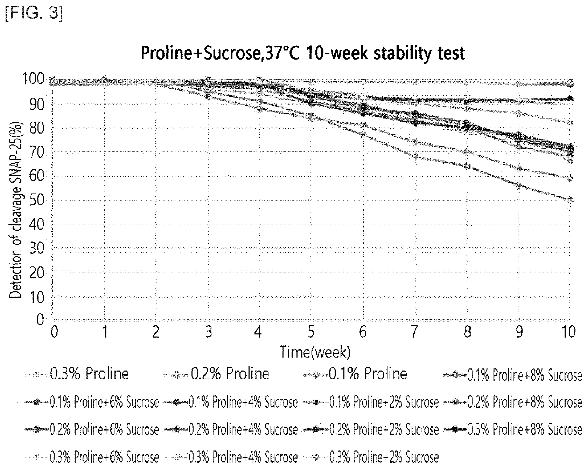

As a result, as shown in Table 3 and FIG. 3, it was confirmed that the combination of 0.2% (w/v) to 0.3% (w/v) proline and 4% (w/v) to 8% (w/v) sucrose showed a residual potency of 90% or more for 10 weeks under 37°C accelerated conditions.

**[Table 3]**

| | 0 wk | 1 wk | 2 wk | 3 wk | 4 wk | 5 wk | 6 wk | 7 wk | 8 wk | 9 wk | 10 wk |
|---|---|---|---|---|---|---|---|---|---|---|---|
| 0.3% Proline | 99 | 99 | 100 | 99 | 99 | 92 | 90 | 82 | 78 | 74 | 69 |
| 0.2% Proline | 100 | 99 | 99 | 96 | 94 | 90 | 90 | 83 | 79 | 76 | 66 |
| 0.1% Proline | 100 | 98 | 98 | 93 | 88 | 84 | 78 | 74 | 70 | 63 | 59 |
| 0.1% Proline+8% Sucrose | 100 | 100 | 99 | 97 | 96 | 91 | 87 | 83 | 80 | 72 | 68 |
| 0.1% Proline+6% Sucrose | 100 | 99 | 100 | 98 | 97 | 94 | 89 | 85 | 81 | 76 | 71 |
| 0.1% Proline+4% Sucrose | 99 | 99 | 100 | 98 | 98 | 93 | 88 | 86 | 82 | 75 | 70 |
| 0.1% Proline+2% Sucrose | 99 | 100 | 100 | 95 | 91 | 85 | 77 | 68 | 64 | 56 | 50 |
| 0.2% Proline+8% Sucrose | 98 | 98 | 98 | 98 | 99 | 96 | 93 | 92 | 92 | 91 | 90 |
| 0.2% Proline+6% Sucrose | 99 | 99 | 99 | 98 | 100 | 99 | 99 | 99 | 99 | 98 | 98 |
| 0.2% Proline+4% Sucrose | 99 | 99 | 99 | 98 | 99 | 95 | 92 | 91 | 91 | 91 | 92 |
| 0.2% Proline+2% Sucrose | 100 | 99 | 99 | 99 | 98 | 90 | 86 | 82 | 80 | 77 | 72 |
| 0.3% Proline+8% Sucrose | 100 | 99 | 100 | 99 | 99 | 94 | 92 | 92 | 91 | 92 | 92 |
| 0.3% Proline+6% Sucrose | 100 | 99 | 100 | 100 | 99 | 96 | 94 | 93 | 93 | 92 | 90 |
| 0.3% Proline+4% Sucrose | 99 | 98 | 98 | 99 | 100 | 99 | 99 | 99 | 99 | 98 | 99 |
| 0.3% Proline+2% Sucrose | 100 | 99 | 99 | 100 | 100 | 95 | 92 | 90 | 88 | 86 | 82 |

### 3-4. Evaluation of residual potency according to combination of rHSA and excipient

In order to examine whether the residual potency was excellent even when rHSA was included, rHSA was further included in addition to the excipient of Example 3-1 to evaluate the residual potency.

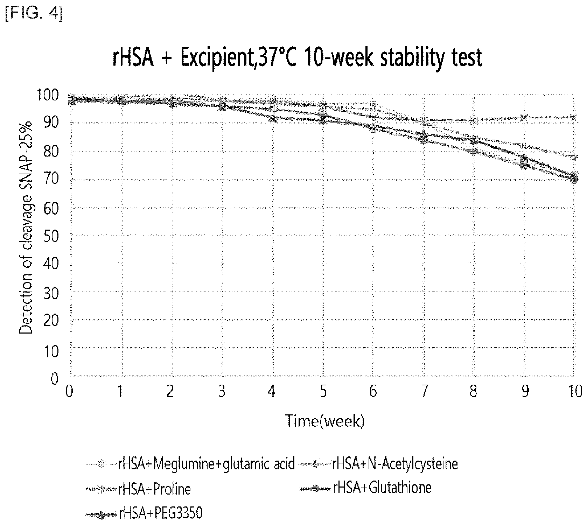

As a result, as shown in FIG. 4, it was confirmed that combination of rHSA and proline showed the most excellent residual potency of 90% or more for 10 weeks under 37°C accelerated conditions.

The above results indicate that the combination of rHSA and proline as the stabilizer in the composition including botulinum toxin may preserve botulinum toxin for a long period of time without reducing its potency in the liquid formulation.

### 3-5. Evaluation of residual potency according to combination of rHSA and disaccharide

Based on the results of Example 3-2 and Example 3-4, it was confirmed that the combination of rHSA and disaccharide maintained stability for a long period of time, and thus liquid formulations were prepared in order to compare residual potencies according to the type of disaccharide.

In detail, sucrose and lactose were used as the disaccharide.

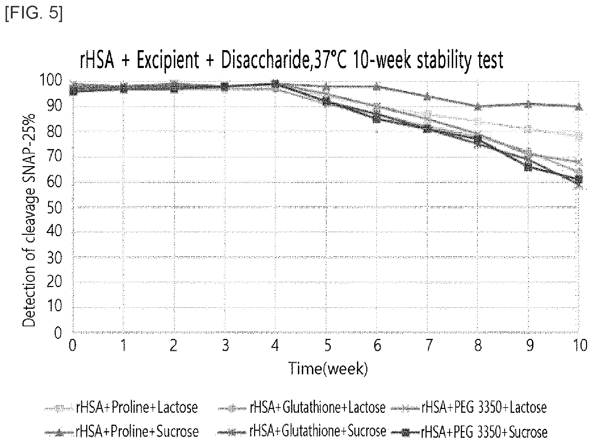

As a result, as shown in FIG. 5, it was confirmed that combination of rHSA, proline, and sucrose showed the most excellent residual potency of 90% or more for 10 weeks under 37°C accelerated conditions.

The above results reaffirmed that the combination of rHSA, proline, and sucrose as the stabilizer in the composition including botulinum toxin may preserve botulinum toxin for a long period of time without reducing its potency in the liquid formulation.

### 3-6. Evaluation of residual potency of liquid formulation including rHSA according to concentration of sucrose

Example 3-5 confirmed that the combination of rHSA, proline, and sucrose was most effective as the stabilizer, and residual potency according to the concentration of proline and sucrose was evaluated.

In detail, 0.1% to 0.3% of proline and 0% to 8% of sucrose were used in the liquid formulation including rHSA.

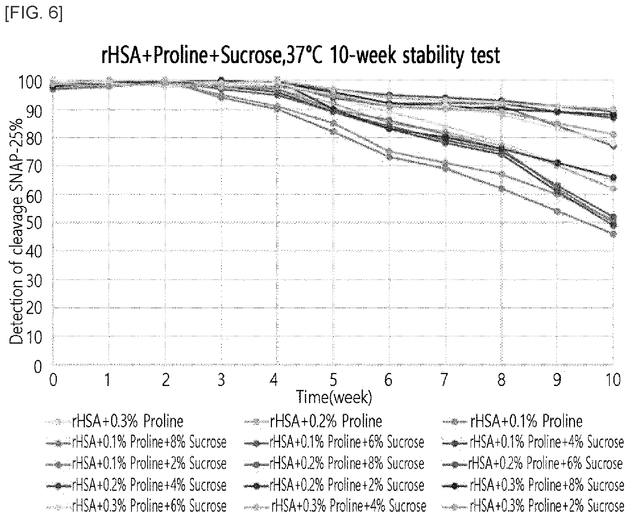

As a result, as shown in Table 4 and FIG. 6, it was confirmed that the combination of 0.2% to 0.3% of proline and 4% to 8% of sucrose showed a residual potency of about 80% or more for 10 weeks under 37°C acceleration conditions.

**[Table 4]**

| | 0 wk | 1 wk | 2 wk | 3 wk | 4 wk | 5 wk | 6 wk | 7 wk | 8 wk | 9 wk | 10 wk |
|---|---|---|---|---|---|---|---|---|---|---|---|
| rHSA+0.3% Proline | 100 | 100 | 99 | 99 | 100 | 94 | 89 | 84 | 78 | 71 | 65 |
| rHSA+0.2% Proline | 100 | 99 | 98 | 98 | 97 | 92 | 85 | 82 | 77 | 70 | 62 |
| rHSA+0.1 % Proline | 100 | 100 | 100 | 95 | 91 | 85 | 75 | 71 | 67 | 60 | 51 |
| rHSA+0.1% Proline+8% Sucrose | 100 | 99 | 100 | 98 | 97 | 90 | 86 | 81 | 76 | 62 | 50 |
| rHSA+0.1% Proline+6% Sucrose | 100 | 100 | 100 | 97 | 95 | 89 | 84 | 79 | 75 | 63 | 52 |
| rHSA+0.1% Proline+4% Sucrose | 100 | 99 | 100 | 98 | 96 | 89 | 83 | 78 | 74 | 61 | 49 |
| rHSA+0.1% Proline+2% Sucrose | 98 | 99 | 100 | 94 | 90 | 82 | 73 | 69 | 62 | 54 | 46 |
| rHSA+0.2% Proline+8% Sucrose | 97 | 98 | 100 | 99 | 99 | 94 | 91 | 90 | 91 | 84 | 77 |
| rHSA+0.2% Proline+6% Sucrose | 100 | 100 | 99 | 98 | 98 | 97 | 95 | 94 | 93 | 91 | 89 |
| rHSA+0.2% Proline+4% Sucrose | 99 | 99 | 98 | 99 | 100 | 96 | 92 | 92 | 92 | 89 | 87 |
| rHSA+0.2% Proline+2% Sucrose | 99 | 99 | 99 | 99 | 100 | 90 | 83 | 80 | 76 | 71 | 66 |
| rHSA+0.3% Proline+8% Sucrose | 98 | 99 | 100 | 100 | 99 | 96 | 92 | 91 | 90 | 89 | 88 |
| rHSA+0.3% Proline+6% Sucrose | 100 | 99 | 98 | 99 | 99 | 95 | 91 | 91 | 88 | 83 | 78 |
| rHSA+0.3% Proline+4% Sucrose | 99 | 100 | 100 | 99 | 100 | 97 | 94 | 93 | 92 | 91 | 90 |
| rHSA+0.3% Proline+2% Sucrose | 99 | 98 | 99 | 98 | 98 | 95 | 91 | 90 | 89 | 85 | 81 |

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

### Effect of the invention

A liquid formulation of botulinum toxin according to the present disclosure is in an immediately applicable form without going through a rehydration process, and therefore, user convenience is improved, it is possible to reduce variations in the botulinum toxin activity due to dilution errors during the rehydration process, and quantitative administration is possible. In addition, the storage stability in the liquid state is superior, and the activity of botulinum toxin is maintained for a long period of time even under room-temperature or refrigerated conditions.

## Claims

1. A liquid formulation comprising botulinum toxin and proline.

2. The liquid formulation of claim 1, wherein the botulinum toxin is botulinum toxin type A.

3. The liquid formulation of claim 1, wherein the proline is included at a concentration of 0.01% (w/v) to 0.5% (w/v), based on the total liquid formulation.

4. The liquid formulation of claim 1, further comprising sucrose.

5. The liquid formulation of claim 4, wherein the sucrose is included at a concentration of 0.5% (w/v) to 20% (w/v), based on the total liquid formulation.

6. The liquid formulation of claim 1, further comprising albumin.

7. The liquid formulation of claim 6, wherein the albumin is recombinant albumin.

8. The liquid formulation of claim 6, wherein the albumin is included at a concentration of 0.01% (w/v) to 1% (w/v), based on the total liquid formulation.

9. The liquid formulation of claim 1, further comprising a non-ionic surfactant and an isotonic agent.

10. The liquid formulation of claim 9, wherein the non-ionic surfactant is one or more selected from the group consisting of polysorbate 20, polysorbate 40, polysorbate 60, polysorbate 80, and polysorbate 100.

11. The liquid formulation of claim 1, further comprising a non-ionic surfactant at a concentration of 0.005% (w/v) to 0.1% (w/v).

12. The liquid formulation of claim 9, wherein the isotonic agent is one or more selected from the group consisting of sodium chloride, glycerin, potassium chloride, and dextrose.

13. The liquid formulation of claim 1, comprising
100 units/mL botulinum toxin; and
0.01% (w/v) to 0.5% (w/v) proline.

14. The liquid formulation of claim 1, comprising
100 units/mL botulinum toxin;
0.01% (w/v) to 0.5% (w/v) proline; and
0.01% (w/v) to 1% (w/v) albumin.

15. The liquid formulation of claim 1, comprising
100 units/mL botulinum toxin;
0.01% (w/v) to 0.5% (w/v) proline; and
0.5% (w/v) to 20% (w/v) sucrose.

16. The liquid formulation of claim 1, comprising
100 units/mL botulinum toxin;
0.01% (w/v) to 0.5% (w/v) proline;
0.01% (w/v) to 1% (w/v) albumin; and
0.5% (w/v) to 20% (w/v) sucrose.

17. A method of stabilizing botulinum toxin using a liquid formulation including botulinum toxin and proline.

18. The method of claim 17, wherein albumin, sucrose, or both albumin and sucrose is/are further included in the liquid formulation.
